# EUROPEAN PATENT APPLICATION

(11) **EP 0 540 462 A1**
(43) Date of publication of application: **05.05.1993**
(21) Application number: 92810752.3
(22) Date of filing: 06.10.1992
(51) Int. Cl.: A61K 31/195, A61K 37/02, A23L 1/305

(54) **L-Glutamine or a peptide rich in L-glutamine for the manufacture of an oral formulation for the treatment of a fall in blood L-glutamine levels**

(30) Priority: 10.10.1991 GB 9121467
(71) Applicant: SANDOZ NUTRITION LTD., CH-3001 Berne (CH)
(72) Inventor: Brouns, Fredericus Johannes Petrus Henricus, CH-3014 Berne (CH); Wagenmakers, Antonius Johannes Maria, NL-6325 PH Berg en Terblyt (NL); Saris, Wilhelmus Hermanus Marinus, NL-6231 KS Meerssen (NL)
(74) Representative: Smolders, Walter

(57) **Abstract**

Compositions for oral administration of L-glutamine, in free amino acid form, or in the form of a physiologically acceptable L-glutamine source, are suitable for treating or preventing a fall in blood L-glutamine levels in a person involved in endurance exercise, physical activity or suffering from overtraining.

## Description

This invention relates to the oral use of L-glutamine, in free amino acid form or in peptide form (hereinafter also referred to as L-glutamine source according to the invention).

It has been suggested that intensive training and overtraining leads to a fall in plasma-L-glutamine concentration and that this fall plays a role in a weakened immune status and increased susceptibility to colds and infections. It is also known that catabolism, resulting in net gut protein breakdown, during intense exercise is increased and that this may be related to impaired gut function during exercise. Exhausting endurance exercise leads furthermore to a substantial fall in the muscle L-glutamine due to a release from exercising muscles and to a fall in plasma glutamine levels either through a reduced L-glutamine production in the muscles or through an increased capture from the blood.

By its action on glycogen synthase in liver and muscle L-glutamine may affect glycogen depletion during exercise and glycogen recovery following exercise. High levels of L-glutamine in muscle increase muscle protein synthesis and decrease muscle protein degradation. L-glutamine is also a fuel for the gut. It furthermore stimulates protein synthesis in rapidly dividing cells such as gut mucosal cells. L-glutamine is also known to be a gluconeogenic (glucose building) precursor.

It is therefore desirable to secure adequate intracellular and blood serum L-glutamine levels during intensive physical work or exercise.

It is however known, that the supply of normal protein sources such as meat, egg or milk which contain only from about 3 to about 9 % by weight of L-glutamine during exercise is unsuitable, since the amount of protein needed to supply a significant amount of L-glutamine would be too high. Higher levels of protein intake during exercise have been observed to enhance blood ammonia levels, which suppress oxidative energy production and induces fatigue.

It has now surprisingly been found that L-glutamine, in free amino acid form, or in the form of a physiologically acceptable L-glutamine source, ingested shortly before and during exercise is not consumed in a significant amount by the gut and therefore significantly enhances blood L-glutamine levels. The invention therefore provides a method for treating or preventing a fall in blood L-glutamine levels in a person involved in endurance exercise, physical activity or suffering from overtraining, comprising orally administering to said person a physiologically effective amount of L-glutamine, in free amino acid form, in the form of peptides rich in L-glutamine or in the form of other physiologically acceptable L-glutamine derivatives; or a mixture thereof.

Where the L-glutamine is supplied in the form of peptides rich in L-glutamine, the L-glutamine content of said peptide should be sufficiently high to secure the beneficial effect of L-glutamine while substantially avoiding the undesirable effects induced by protein supply. The peptides to be employed in the method of the invention therefore conveniently contain at least 20 % by weight, preferably more than 24 % by weight of L-glutamine. Suitable peptide sources for L-glutamine include short chain peptides such as dipeptides, e.g. L-alanyl-L-glutamine (which contains ca. 63 % by weight of L-glutamine), tripeptides, tetrapeptides, pentapeptides, hexapeptides, longer chain peptides or peptide mixtures, e.g. in the form of isolated proteins rich in L-glutamine, hydrolysates of L-glutamine rich proteins or appropriate fraction of such proteins or hydrolysates. Typical examples of L-glutamine rich protein sources are for example wheat (which is a source for gliadin, a protein containing 35 to 43 % of glutamine) and beans (of which the seed storage protein contains ca. 24 % of glutamine).

According to a preferred embodiment the peptides rich in L-glutamine are substantially in short chain form and contain from 2 to 10, preferably from 2 to 8, more preferably from 2 to 6, particularly from 2 to 4 amino acids.

Other physiologically acceptable L-glutamine derivatives are known in the art. They include L-glutamine salts, N-acyl derivatives of L-glutamine including N-alkanoyl L-glutamine compounds such as N-acetyl L-glutamine. The N-acylation of L-glutamine stabilises, and therefore prevents decomposition of L-glutamine to L-pyroglutamate. Such "other physiologically acceptable L-glutamine derivatives" will conveniently also be rich in L-glutamine, i.e. contain at least 20 % by weight, preferably more than 24 % by weight of L-glutamine.

The supply of L-glutamine, in free amino acid or in the form of a physiologically acceptable L-glutamine source does not significantly enhance blood ammonia levels, nor does it decrease performance if said L-glutamine source is sufficiently rich in L-glutamine; it also, when added to a drink, does not inhibit gastric emptying to a physiologically significant degree, and accordingly secures maximum fluid and nutrient availability. The L-glutamine, in free amino acid or in the form of a physiologically acceptable L-glutamine source according to the invention, can be ingested alone or in the form of a pharmacologically or nutritionally acceptable formulation form.

Depending on the particular use contemplated, the formulated forms may include nutritional elements such as vitamins, minerals including trace elements and/or energy sources. The L-glutamine or L-glutamine source according to the invention, may be formulated in any form suitable for oral administration, i.e. in solid or liquid form, or in a concentrate form intended for dilution prior to ingestion. It may be formulated employing conventional formulation ingredients such as binding agents, diluents, flavouring agents and the like. The administration form employed will depend on the particular purpose or circumstances of the ingestion. Thus, the L-glutamine or L-glutamine source according to the invention, may be formulated in the form of a nutritional supplement, e.g. in an appropriate galenic - solid or liquid - form for such as a tablet, a capsule or a syrup. It may be formulated as a thirst quencher or a thirst quencher concentrate. It may be combined with appropriate energy sources to secure adequate energy supply e.g. in the form of an energy bar or be combined with appropriate minerals and energy sources for rehydration and energy supply.

Preferably the formulation is in liquid form and suitable for drinking or in a dry or otherwise concentrate form which is suitable for dissolution in water.

The daily intake of L-glutamine in free amino acid form, or in the form of a physiologically acceptable L-glutamine source should conveniently not exceed 1.0 g/kg body weight. For adults involved in stressful physiological conditions the daily amount to be administered will conveniently lie in the range of from 0.1 to 70 g, preferably of 0.5 to 50 g of L-glutamine. For use according to the invention in a liquid form suitable concentrations of L-glutamine, in free amino acid form or in the form of a physiologically acceptable L-glutamine source according to the invention or of a mixture thereof, will conveniently lie in the range of from 0.01 g to 50 g, preferably of from 5 g to 50 g, more preferably of from 10 to 30 g per litre. In case the drink is a sport drink intended for rehydration and energy supply, the L-glutamine will conveniently be ingested in more dilute form. Such sport drinks will conveniently comprise from 2 g to 10 g, e.g. 6 g of L-glutamine in free amino acid form or in the form of a physiologically acceptable L-glutamine source according to the invention or of a mixture thereof per litre. Where the drink is intended for use as a nutritional supplement, it will conveniently contain 2 g to 10 g per serving of 100 ml to 300 ml.

Energy sources suitable for use in formulations of the invention, will prevent blood glucose to drop to low levels and initiate sparing of endogenous carbohydrate pools.

Examples of such energy sources include carbohydrates, such as glucose, dextrose, fructose, maltose, sucrose, lactose, maltotriose, glucose polymers, malto-dextrins, dextrins, starch and soluble starch; fat, such as medium chain triglycerides (MCT). The term MCT as used herein refers to glycerin triesters with fatty acids having a carbon chain length of C6 to C12. Where a fat energy source is present, it is preferably in MCT form. The relative proportion of L-glutamine to the energy delivering nutrients can vary within wide ranges as long as there is no significant delay in gastric emptying, when added to a drink, and no increase in blood ammonia levels during exercise.

Where the L-glutamine and/or L-glutamine source according to the invention is ingested in combination with an energy source, the weight ratio of L-glutamine and L-glutamine source: energy sources free of L-glutamine will conveniently vary within the range of from 1:0.25 to 1:20. This is particularly so if the energy sources employed are primarily carbohydrates. Where the energy source employed comprises also a substantial proportion of fat, said weight ratio is preferably within the range of from 1:0.5 to 1:14.

The formulations according to the invention are preferably substantially free of proteins containing less than 20 %, more preferably less than 24 % by weight of L-glutamine. Preferably the nitrogen sources present in the formulation of the invention, contain at least 20 % by weight, more preferably more than 24 % by weight of L-glutamine.

The formulations suitable for use in the method of the invention, conveniently contain sodium to enhance absorption or other electrolytes to replace those lost with sweating. The electrolyte content of rehydration drinks containing the L-glutamine source according to the invention, is preferably selected such that the drink solution does not comprise more than 2000 mg/liter chloride, 2000 mg/liter sodium, 400 mg/liter potassium, 300 mg/liter calcium and 200 mg/liter magnesium.

In rehydration drinks (thirst quenchers), the electrolyte content should more desirably not exceed the following levels (the preferred range is indicated between brackets):

| | | |
|---|---|---|
| - chloride: | 1500 mg/liter | (500 to 1500 mg/l) |
| - sodium: | 1100 mg/liter | (400 to 1100 mg/l) |
| - potassium: | 225 mg/liter | (120 to 225 mg/l) |
| - calcium: | 225 mg/liter | (45 to 225 mg/l) |
| - magnesium: | 100 mg/liter | (10 to 100 mg/l). |

Any vitamins added to the formulation for use in the method of the invention are preferably in amounts according to national food legislation.

Since L-glutamine in free amino acid form is unstable in liquids, the compound is preferably employed in peptide form, salt form of N-acylated form.

The dipeptide L-alanyl-L-glutamine is particularly appropriate for use in the method of the invention in view of its excellent stability in solution and during heat sterilisation. It has a solubility of about 500 gram/liter in water and it is nearly tasteless. L-Alanine is, as L-glutamine a gluconeogenic precursor. L-Alanyl-L-glutamine is for all these reasons particularly suitable for use as a supplement to existing sport drinks.

The following examples illustrate the invention.

### EXAMPLE

| Composition per liter solution | |
|---|---|
| Carbohydrate | 40 g |
| Fat (in MCT form) | 5 g |
| Protein | -- |
| L-Alanyl-L-glutamine | 10 g |
| Sodium | 600 mg |
| Potassium | 150 mg |
| Chloride | 600 mg |
| Magnesium | 100 mg |
| Calcium | 100 mg |
| Vitamins | 150 mg |

### Experiment 1:

### Methods

Seven volunteers (5 male, 2 female) first lowered their muscle glycogen concentration by an exercise protocol at alternating intensities. After warming up they cycled in 2-min blocks alternating at 90 and 50 % Wmax. This was continued until the 2-min block at 90 % Wmax could not be completed anymore. The high intensity was then reduced to 80 % until it could not be completed anymore and finally reduced to 70 % Wmax. When the 70 % Wmax block could not be completed, the subjects were allowed to stop and rest. 30 Min later L-glutamine (pyrogen free infusion quality, Degussa AG, HANAU, FRG; 0.35 g/kg body weight) was taken, orally suspended in skimmed yoghurt (2 g/kg body weight) and mixed with a low carbohydrate raspberry syrup (1 g/kg body weight). The total carbohydrate content of this mix was 0.09 g/kg body weight. In the control test only yoghurt mixed with raspberry syrup was taken. Cycling exercise was resumed 90 min later: 5 min at 50 % Wmax followed by 30 min at 65 % Wmax and finally until exhaustion at 75 % Wmax. Blood was sampled from a forearm venous cannula and plasma analysed for amino acids (HPLC) and ammonia (enzymatic assay with glutamate dehydrogenase). Heart rate was monitored each 5 min.

### Results

The glutamine supplement marginally increased plasma ammonia immediately before and 10 min into the second exercise bout. No significant differences were present during the second part of the exercise test and at exhaustion. Heart rate was not different between the test during exercise and at exhaustion. Plasma glutamine concentration was significantly higher after glutamine supplementation both before and after the second exercise bout. Glutamine supplementation did not influence time to exhaustion.

### Experiment 2

### Methods

Six male volunteers were studied during two cycling exercise tests performed at random order 90 min following a standardised breakfast (6 ml/kg body weight of Meritene^{R} and 2 slices of white bread). The protocol was as follows: 10 min at 30 % Wₘₐₓ followed by 10 min at 50 % Wmax (warm-up); then eight periods of alternating exercise first 6 min at 60 % Wₘₐₓ followed by 6 min at 80 % Wₘₐₓ. Finally the subjects exercised to exhaustion at 90 % Wₘₐₓ. In one of the tests the subject drank a carbohydrate/electrolyte solution (Isostar^{R}), in the other Isostar^{R} supplemented with 20 gram/L of L-alanyl-L-glutamine (pyrogen free infusion quality, Degussa AG, Hanau, FRG). Drinks were taken as a bolus after 10 min warm-up and then as repeated bolusses of 300 ml each 30 min. Blood was taken on one occasion before exercise (rest sample) and at exhaustion in both tests and plasma was analysed for amino acids (HPLC).

### Results

L-Alanyl-L-glutamine supplementation increased both alanine and glutamine concentration above the rest value (before intake started) and above the control test in which only Isostar^{R} was ingested. Subjects did not notice much of a taste deference between Isostar^{R} and Isostar^{R} containing L-alanyl-L-glutamine. L-Alanyl-L-glutamine supplementation did not influence time to exhaustion during the final exercise bout (8-10 min) at 90 % Wₘₐₓ.

## Claims

1. The use of L-glutamine in free amino acid form, or in the form of a peptide rich in L-glutamine, or in the form of another physiologically acceptable L-glutamine derivative, or of a mixture thereof for the manufacture of an oral administration form or a concentrate thereof for the treatment or prevention of a fall in the blood L-glutamine level in people involved in endurance exercise, physical activity or suffering from overtraining.

2. The use according to Claim 1, for the manufacture of a nutritional supplement or a concentrate thereof, for supply to a person involved in endurance exercise, physical activity or suffering from overtraining.

3. The use according to Claim 2, whereby the nutritional supplement or nutritional supplement concentrate is in the form of a tablet, a capsule, a thirst quencher or a concentrate thereof.

4. The use according to Claim 1, for the manufacture of an energy bar, a sport drink or a sport drink concentrate for energy supply to a person involved in endurance exercise, physical activity or suffering from overtraining.

5. An oral formulation for the treatment or prevention of a fall in the blood L-glutamine level in people involved in endurance exercise, physical activity or suffering from overtraining comprising a physiologically effective amount of L-glutamine, in free amino acid form, or in the form of a peptide rich in L-glutamine, or in the form of another physiologically acceptable L-glutamine derivative, or of a mixture thereof.

6. The formulation of Claim 5 comprising energy sources in a weight ratio of L-glutamine and L-glutamine source : energy sources free of L-glutamine in the range of from 1:0.25 to 1:20.

7. The oral formulation of Claim 6, comprising a substantial proportion of fat as an energy source, whereby the weight ratio L-glutamine source: energy source is within the range of from 1:0.5 to 1:14.

8. The formulation of Claims 5 to 7, which is substantially free of' peptides containing less than 20 % by weight of L-glutamine.

9. The formulation of Claims 5 or 8 in the form of a tablet, a capsule, a thirst quencher or a concentrate thereof.

10. The formulation of Claims 5 to 8 in the form of a solid or liquid energy supplement or of a concentrate thereof.

11. The formulation of Claims 5 to 10 in drink form or in a concentrate form suitable for dissolution in water to a drinkable form.

12. The formulation according to Claim 11 in drink form or which after dissolution in water gives a drinkable form comprising from 0.01 g to 50 g of L-glutamine in free amino acid form, or in the form of a peptide rich in L-glutamine, or of another physiologically acceptable L-glutamine derivative, or of a mixture thereof per litre of drink.

13. The composition of Claim 5 to 12 containing fat in the form of medium chain triglycerides.

14. The composition of Claims 5 to 13, wherein the L-glutamine is in the form of peptides rich in L-glutamine.

15. The composition of Claim 14, wherein the L-glutamine source contains at least 20 % by weight of L-glutamine.

16. The composition of Claim 15, wherein the L-glutamine source consists substantially of short chain peptides.

17. The composition of Claim 16, wherein the L-glutamine is in the form of L-alanyl-L-glutamine.
